# EUROPÄISCHE PATENTSCHRIFT

(11) **EP 1 136 822 B1**
(45) Veröffentlichungstag und Bekanntmachung des Hinweises auf die Patenterteilung: **22.06.2005**
(21) Anmeldenummer: 01106571.1
(22) Anmeldetag: 15.03.2001
(51) Int. Cl.: G01N 33/68, G01N 1/30

(54) **Verfahren zur Identifizierung von zellspezifischen Zielstrukturen**
Method for identifying cell specific target structures
Méthode pour l'identification des structures cibles spécifiques aux cellules

(30) Priorität: 24.03.2000 DE 10014685
(43) Veröffentlichungstag der Anmeldung: 26.09.2001
(73) Patentinhaber: MPB MelTec Patent- und Beteiligungsgesellschaft mbH, 39120 Magdeburg (DE)
(72) Erfinder: Schubert, Walter, Dr., 39175 Biederitz (DE)
(74) Vertreter: Schurack, Eduard F.

(56) Entgegenhaltungen:
- EP-A- 0 701 130
- WO-A-93/18068
- WO-A-95/34817
- DE-A- 19 709 348
- US-A- 5 437 987
- WYSOCKI L J ET AL: "PANNING FOR LYMPHOCYTES A METHOD FOR CELL SELECTION" PROCEEDINGS OF THE NATIONAL ACADEMY OF SCIENCES OF THE UNITED STATES, Bd. 75, Nr. 6, 1978, Seiten 2844-2848, XP002901409 1978 EN ISSN: 0027-8424
- SCHUBERT WALTER ET AL: "Detection by 4-parameter microscopic imaging and increase of rare mononuclear blood leukocyte types expressing the Fc-gamma-RIII receptor (CD16) for immunoglobulin G in human sporadic amyotrophic lateral sclerosis (ALS)." NEUROSCIENCE LETTERS, Bd. 198, Nr. 1, 1995, Seiten 29-32, XP000983323 ISSN: 0304-3940

## Beschreibung

Die Erfindung betrifft ein Verfahren zur Identifizierung von zellspezifischen Zielstrukturen.

Die Identifizierung zellspezifischer Zielstrukturen ist von zentraler Bedeutung bei der Aufklärung von Zell-Zell-Interaktionen, die unzählige Wirkungen innerhalb eines Organismus nach sich ziehen können. Insbesondere die Kenntnis krankheitsspezifische Zielstrukturen ist eine entscheidende Voraussetzung für die Entwicklung wirksamer und gleichzeitig nebenwirkungsarmer Arzneimittel.

Es ist bekannt, daß Immunzellen (Lymphozyten) spezifische Kombinationen von Proteinen, auch Proteinkombinationsmuster bzw. kurz PKM genannt, exprimieren, die für eine Bindung an Endothelzellen der Blutgefäße von Gehirn und Muskelgewebe verantwortlich sind. Andere Kombinationen von Proteinen führen dagegen nicht zu einer Bindung an diese Endothelzellen. Überraschenderweise sind diese spezifischen Kombinationen interindividuell konstant und weisen immer dieselben Bindungsfunktionen auf. Es scheint sich folglich bei den spezifischen Proteinkombinationsmustern um einen interindividuellen konstanten Lymphozyten Bindungs-Code der Zelloberfläche für organspezifische Endothelzelloberflächen zu handeln, der eine zellspezifische Zielstruktur darstellt. Zellspezifische Zielstrukturen können folglich ganz spezifische Proteinkombinationsmuster aufweisen.

Auch invasive Tumorzellen verfügen über spezifische Proteinkombinationsmuster an ihren Zelloberflächen, die zu einem gezielten, d.h. organselektiven Invasionsverhalten führen. Solche Proteinkombinationsmuster stellen daher Zielstrukturen für mögliche Arzneimittel dar.

Unbedingte Voraussetzung für die Entwicklung solcher hoch-selektiver Arzneimittel ist jedoch die Kenntnis der molekularen Zusammensetzung dieser Zielstrukturen.

Aus dem Stand der Technik sind Verfahren zur Identifizierung von Zielstrukturen bekannt, die auf der Analyse von Gen-Expressionsprofilen von kranken Geweben oder Zellen im Vergleich zu Gen-Expressionsprofilen von gesunden Geweben oder Zellen beruhen, wobei sowohl Proteinexpressionsprofile als auch Expressionsprofile der Messenger Ribonukleinsäure (mRNA) Aufschluß über das Auftreten neuer Proteine, fehlregulierter oder abnorm modifizierter Proteine in kranken Geweben oder Zellen geben sollen (z.B. in: F. Lottspeich/H.Zorbas; Bioanalytik; Spektrum Akademischer Verlag; Heidelberg, 1998).

Diese Verfahren gehen jedoch alle von Zellhomogenaten aus, denen in der Regel Tausende oder Millionen von Zellen zugrunde liegen, da nur mit Hilfe dieser Zellmengen Expressionsprofile der oben genannten Art erstellt werden können. In den Zellhomogenaten liegen die Zellen in aufgeschlossener Form vor, damit die Proteine oder mRNA-Moleküle mit Hilfe biochemischer Verfahren extrahiert und getrennt werden können.

Nachteilig an diesen bekannten Verfahren ist jedoch, daß sie nicht dazu geeignet sind, Proteinkombinationsmuster zu identifizieren, da die einzelnen Proteinkomponenten eines solchen Proteinkombinationsmusters durch die Erzeugung von Zellhomogenaten und durch die darauffolgenden Extraktionsvorgänge vollständig getrennt werden und die wesentliche Information bezüglich ihrer zell- und gewebetopologischen Lage verloren geht. Durch die Zerstörung der Zellkompartimente können weiterhin keine Informationen bezüglich der Kombinationen von Proteinen innerhalb dieser Zellkompartimente und ihre relative topologische Beziehung zueinander gewonnen werden.

Ein weiterer Nachteil der bekannten Verfahren ist außerdem, daß keine Analytik auf dem Einzelzellniveau durchführbar ist, so daß Unterschiede der einzelnen Zellen bezüglich ihrer Proteinkombinationsmuster nicht erfaßbar sind. Außerdem können Proteine, die nur in geringer Menge vorliegen, durch die bekannten Verfahren nicht detektiert werden. Dies betrifft insbesondere Proteine oder spezifische Proteinkombinationen, die beispielsweise nur in wenigen, jedoch pathogenen, krankheitsspezifischen Zellen vorkommmen.

Ein weiterer Nachteil der bekannten Verfahren besteht darin, daß die Präparationsschritte des Gewebes oder der Zellen von ihrer Entnahme oder Gewinnung bis zum Schritt der Isolierung bzw. Auftrennung von Proteinen einer großen Zahl von variablen externen Einflüssen unterliegen können, die nur schwer kontrollierbar und zu vereinheitlichen sind. Ein automatisiertes Verfahren zur bestimmung einer beliebigen Zahl an Molekülklassen, - teilen oder Gruppen in Objekten und eine Vorrichtung dafür wird in DE-A-19709348 beschrieben.

Aufgabe der Erfindung ist es daher ein Verfahren der oben genannten Art bereitzustellen, durch das zellspezifische Proteinkombinationsmuster identifiziert werden können und durch das die aufgeführten Nachteile des Stands der Technik überwunden werden.

Diese Aufgabe wird gelöst durch ein erfindungsgemäßes Verfahren zur Identifizierung von zellspezifischen Zielstrukturen, das folgende Schritte umfaßt: (a) automatisiertes Aufbringen einer Reagenzlösung Y1, die mindestens ein Markiermolekül aufweist, auf ein Objekt X1, das Zellen und/oder Zellmembranen aufweist, die einer Zell- oder Gewebeprobe entstammen; (b) Einwirken der Reagenzlösung Y1 und automatisierte Detektion mindestens eines Markierungsmusters des mit der Reagenzlösung Y1 markierten Objektes X1; (c) Entfernen der Reagenzlösung Y1 vor oder nach der Detektion des Markierungsmusters und Wiederholung der Schritte a) und b) mit weiteren Reagenzlösungen Yn (n= 2, 3...N), die jeweils das mindestens eine Markiermolekül und/oder mindestens ein anderes Markiermolekül aufweisen; (d) Zusammenfassen der jeweils in Schritt b) detektierten Markierungsmuster zu einem komplexen molekularen Kombinationsmuster des Objekts X1; (e) Wiederholung der Schritte a) bis d) mit mindestens einem weiteren Objekt Xn (n= 2, 3...N), das andere Zellen und/oder andere Zellmembranen aufweist, die einer anderen Zell- oder Gewebeprobe entstammen; (f) Ermitteln mindestens eines Unterschieds zwischen dem Kombinationsmuster des Objektes X1 und dem Kombinationsmuster des Objektes Xn; (g) Identifizierung mindestens einer Reagenzlösung Y1 oder Yn, deren Markierungsmuster den in Schritt f) ermittelten mindestens einen Unterschied verursacht; und (h) Selektion von Molekülen oder Molekülkomplexen, die durch das mindestens eine Markiermolekül der in Schritt g) identifizierten Reagenzlösung Y1 oder Yn gebunden werden, aus einem Homogenat von Zellen und/oder Zellmembranen, die der Zell- oder Gewebeprobe des sich gemäß Schritt f) unterscheidenden Objekts Xn entstammen; und (i) biochemisches Charakterisieren der gemäß Schritt h) selektierten Moleküle oder Molekülkomplexe.

Durch das erfindungsgemäße Verfahren können die Proteinkombinationsmuster einzelner Zellen oder Zellmembranen von unterschiedlichen Zell- oder Gewebeproben vergleichend untersucht werden. Dabei können diejenigen Markiermoleküle identifiziert werden, die beispielsweise an ein bestimmtes Proteinkombinationsmuster oder an einen bestimmten Bereich eines solchen Proteinkombinationsmusters eines ersten Objektes binden, das einer ersten Gewebe- oder Zellprobe entstammt, und die gleichzeitig nicht an ein zweites Objekt binden, das einer zweiten Gewebe- oder Zellprobe entstammt. Mit Hilfe dieser identifizierten Markiermoleküle können nun unter Verwendung eines Probenanteils der ersten Gewebe- und/oder Zellprobe diejenigen molekularen Bereiche (Moleküle oder Molekülkomplexe) des Proteinkombinationsmusters aufgefunden bzw. selektiert und anschließend charakterisiert werden, die durch die identifizierten Markiermoleküle gebunden werden. Auf diese Weise ist es möglich, sowohl die molekulare Zusammensetzung eines Proteinkombinationsmusters, die Anordnung der Moleküle innerhalb des Proteinkombinationsmusters und die Anordnung des Proteinkombinationsmusters innerhalb eines Gewebes oder eine Zelle zu erfassen.

Eine besonders vorteilhafte Weiterbildung der Erfindung sieht vor, daß das in Schritt h) verwendete Homogenat vor Schritt h) durch Molekül- oder Molekülkomplextrennverfahren, insbesondere Proteintrennverfahren, in einzelne Homogenatbestandteile aufgetrennt wird. Auf diese Weise wird die Selektion der Moleküle oder Molekülkomplexe aus dem Homogenat außerordentlich vereinfacht.

Eine weitere vorteilhafte Weiterbildung des erfindungsgemäßen Verfahrens sieht vor, daß das Objekt X1 Zellen und/oder Zellmembranen aufweist, die einer Zell- oder Gewebeprobe eines kranken Patienten entstammen, und daß mindestens ein anderes Objekt Xn Zellen und/oder Zellmembranen aufweist, die einer Zell- oder Gewebeprobe eines gesunden Probanden entstammen. So können krankheitsspezifische Zielstrukturen bzw. die entsprechenden Proteinkombinationsmuster identifiziert werden, so daß aufgrund der Kenntnis dieser krankheitsspezifischen Proteinkombinationsmuster hoch-spezifische Arzneimittel entwickelt werden können, die aufgrund eben dieser Spezifität nahezu nebenwirkungsfrei sind.

Eine weitere vorteilhafte Weiterbildung der Erfindung sieht vor, daß das Verfahren folgenden parallelen Schritt umfaßt: (x) Erstellen jeweils eines Proteinexpressionsprofils von jeweils einem Probenanteil der Zell- oder Gewebeproben, von denen Zellen und/oder Zellmembranen in die Objekte X1 und Xn eingehen, und Vergleichen des dem Objekt X1 zuzuordnenden Proteinexpressionsprofils mit dem dem Objekt Xn zuzuordnenden Proteinexpressionsprofil, wobei mindestens ein Unterschied festgestellt wird.

Weiterhin kann das erfindungsgemäße Verfahren nach Schritt x) folgenden Schritt umfassen: (y) Überprüfen mindestens eines Proteins und/oder mindestens einer Proteinmodifikation, das oder die den in Schritt x) ermittelten Unterschied verursacht, bezüglich einer Bindung des mindestens einen Markiermoleküls der in Schritt g) identifizierten Reagenzlösung Y1 oder Yn. Auf diese Weise kann ermittelt werden, ob es sich bei den durch Vergleich der Proteinexpressionsprofile festgestellten Unterschiede um verfahrensbedingte Artefakte (siehe oben) oder um tatsächlich signifikante Unterschiede handelt, da sie auch durch das erfindungsgemäße Verfahren ermittelt werden konnten. Das erfindungsgemäße Verfahren kann folglich auch als wesentliche Ergänzung zu den bereits bekannten Verfahren verwendet werden.

In einer weiteren besonders vorteilhaften Weiterbildung der Erfindung ist vorgesehen, daß mindestens ein in Schritt a) und/oder Schritt c) verwendetes Markiermolekül mindestens ein Protein und/oder mindestens eine Proteinmodifikation bindet, das oder die den in Schritt x) ermittelten Unterschied verursacht. Durch bekannte Verfahren können beispielsweise Antikörper oder Liganden entwickelt werden, die Proteine oder Proteinmodifikationen binden, die durch den Vergleich von Proteinexpressionsprofilen als Bereiche von Zielstrukturen ermittelt wurden. Durch den Einsatz dieser Antikörper oder Liganden in dem erfindungsgemäßen Verfahren kann überprüft werden, ob die durch den Vergleich von Proteinexpressionsprofilen ermittelten Proteine Proteinkombinationsmuster erzeugen oder nicht.

Weiterhin kann mindestens ein in Schritt a) und/oder Schritt c) verwendetes Markiermolekül Fluorochrom-konjugiert sein. Durch eine solche Fluoreszenzmarkierung ist ein Markiermolekül besonders leicht detektierbar.

Weiterhin kann mindestens ein in Schritt a) und/oder Schritt c) verwendetes Markiermolekül ein Antikörper sein, wobei der Antikörper einer Antikörperbibliothek entnommen werden kann, wobei die Antikörperbibliothek eine naive oder eine nicht-naive Antikörperbibliothek sein kann. Bei einer sogenannten naiven Antikörper-Bibliothek handelt es sich um eine Bibliothek, deren Antikörper keine bekannte Spezifität aufweisen, während die Antikörper der nicht-naiven Antikörperbibliothek bekannte Moleküle, wie Proteine oder Glykoproteine erkennen. Durch Verwendung einer nicht-naiven Bibliothek ist also gleich bei Identifizierung eines Antikörpers eine Information dazu vorhanden, welches Molekül oder welche Moleküle der Antikörper bindet.

Weiterhin kann mindestens ein in Schritt a) und/oder Schritt c) verwendetes Markiermolekül ein Ligand sein. Der Ligand kann einer Ligandenbibliothek entnommen werden, wobei die Ligandenbibliothek eine naive oder eine nicht-naive Ligandenbibliothek sein kann. Die Bezeichnungen "naiv" und "nicht-naiv" sind in Analogie zu den obigen Ausführungen zu verstehen.

Nach dem Entfernen der Reagenzlösung gemäß Schritt c) kann ein Waschschritt folgen, in dem eine Waschlösung auf das Objekt X1 aufgebracht und nach einer vorbestimmten Zeit wieder entfernt wird. Auf diese Weise wird ausgeschlossenen, daß eine neu aufgebrachte Reagenzlösung durch eine vorher aufgebrachte Reagenzlösung verunreinigt wird.

Schritt d) kann in vorteilhafter Weise durch computergestützte Bildüberlagerungen erfolgen.

Das Verfahren kann beliebig oft wiederholbare Bleichungszyklen, insbesondere nach Schritt b), umfassen. Durch solche Bleichungszyklen wird verhindert, daß Markierungen, die bereits detektiert wurden, in einem nachfolgenden Schritt erneut detektiert werden.

Weitere Einzelheiten, Merkmale und Vorteile der Erfindung ergeben sich aus der folgenden Beschreibung eines Ausführungsbeispiels.

In dem Ausführungsbeispiel wird eine Gewebe- oder Zellprobe in zwei Probenanteile unterteilt. Der erste Probenanteil dient als Ausgangsmaterial für die Herstellung eines Homogenats zur biochemischen Proteincharakterisierung nach den bekannten Verfahren. Insbesondere werden Verfahren, wie die 2D-Gelelektrophorese, zur Erstellung von Proteinexpressionsprofilen angewendet.

Der zweite Probenanteil wird zur Herstellung von Gewebeschnitten bzw. von Zellpräparaten mit intakten Zellen verwendet. Handelt es sich bei dem zweiten Probenanteil um einen Gewebeblock, so wird von diesem ein Gewebeschnitt hergestellt. Falls es sich hingegen um Zellen in einer Suspension handelt, werden diese Zellen in intakter Form auf eine Oberfläche, insbesondere auf einen Objektträger aufgebracht. Das auf diese Weise erhaltene erste Objekt wird den folgenden automatisierten Verfahrenschritten unterworfen:
1. Aufnahme einer ersten Reagenzlösung Y1 mit ein oder mehreren fluorochromkonjugierten Antikörpern einer nicht-naiven Antikörperbibliothek;
2. Aufpipettieren dieser Reagenzlösung Y1 auf das erste Objekt;
3. Inkubation des Objekts mit dieser Lösung Y1 bei einer bestimmten Temperatur, insbesondere Raumtemperatur;
4. Entfernen dieser Reagenzlösung;
5. Einmaliges oder mehrmaliges Auftropfen einer Waschlösung und anschließendes Entfernen;
6. Aufbringen einer Pufferlösung auf das erste Objekt;
7. Detektion des Fluoreszenzverteilungsmusters, wobei bei Verwendung von mehreren Fluorochromen selektive Fluoreszenzaufinahmefilter bei der Bildaufnahme verwendet werden. In diesem Schritt wird festgestellt, ob und an welchen Orten der oder die Antikörper in der Probe Bindungssignale zeigen. Es werden Positiv-Signale und Negativ-Signale gleichermaßen für jeden Bildpunkt bzw. Ortspunkt der Zellen oder des Gewebes des ersten Objektes digital registriert;
8. Aufpipettieren einer weiteren Waschlösung;
9. Bleichen der Probe mit Hilfe einer Fluoreszenzanregung, wobei der Bleichungsvorgang dann beendet ist, wenn keine Fluoreszenz mehr detektiert werden kann;
10. Entfernen der weiteren Waschlösung;
11. Aufnahme einer Reagenzlösung Y2 mit einem oder mehreren ebenfalls fluorochromkonjugierten Antikörpern einer anderen oder der gleichen Antikörperbibliothek;
12. Aufpipettieren der Reagenzlösung Y2 auf das erste Objekt.

Dieses Verfahren wird durch Wiederholung der Schritte 1 bis 10 fortgesetzt, wobei die Reagenzlösungen Y3, Y4, Y5..Yn verwendet werden, die Antikörper oder Liganden von nicht-naiven oder naiven Bibliotheken enthalten können. Ein- und dasselbe Objekt kann auf diese Weise mit einer komplett naiven Bibliothek oder mit einer komplett nicht-naiven Bibliothek, oder mit einer gemischt nicht-naiven und naiven Bibliothek untersucht werden.

Jeder dieser Verfahrenszyklen (Schritte 1 bis 10) wird durch Registrierung eines korrespondierenden Phasenkontrastbildes oder Differentialinterferenzkontrastbildes beendet.

Aus den jeweils in Schritt 7 registrierten Markierungsmustern werden die existierenden Signalkombinationen und die nicht existierenden Signalkombinationen, die gesamtheitlich ein Kombinationsmuster ergeben, durch computergesteuerte Bildüberlagerungen ermittelt.

Dieses für das erste Objekt ermittelte Kombinationsmuster wird mit anderen Kombinationsmustern von anderen Proben bzw. Probanden, Zellen oder Zuständen oder Krankheiten verglichen, die mit denselben Reagenzlösungen nach denselben standardisierten oben aufgeführten Verfahrensschritten analysiert wurden.

Sollten bei diesem Vergleich eindeutige, für die untersuchte Probe oder die für einen Zustand, eine Zellform, eine Krankheit oder eine Funktion spezifische Signalkombinationen resultieren, beruhen diese Signalkombinationen auf spezifischen, d.h. selektiven molekularen Interaktionen und charakterisieren daher den Zustand, die Krankheit, die Funktion oder die Zellform. Den ermittelten Signalkombinationen können die entsprechenden Antikörper oder Liganden zugeordnet werden.

Die auf diese Weise herausgefilterten Antikörper und/oder Liganden werden nunmehr dazu eingesetzt, um in dem ersten Probenanteil diejenigen Proteine, Glycoproteine oder andere Kohlenhydratstrukturen "herauszufischen", die diese Liganden oder Antikörper spezifisch binden können. Dazu werden die in der biochemischen Analytik bekannten Verfahren eingesetzt. Die so gefundenen Moleküle können einzelne Molekülspezies oder Komplexe aus verschiedenen Molekülspezies sein. In beiden Fällen stellen sie hoch spezifische Zielstrukturen, insbesondere für die Entwicklung von Arzneimitteln dar.

## Patentansprüche

1. Verfahren zur Identifizierung von zellspezifischen Zielstrukturen, folgende Schritte umfassend
a) automatisiertes Aufbringen einer Reagenzlösung Y1, die mindestens ein Markiermolekül aufweist, auf ein Objekt X1, das Zellen und/oder Zellmembranen aufweist, die einer Zell- oder Gewebeprobe entstammen;
b) Einwirken der Reagenzlösung Y1 und automatisierte Detektion mindestens eines Markierungsmusters des mit der Reagenzlösung Y1 markierten Objektes X1;
c) Entfernen der Reagenzlösung Y1 vor oder nach der Detektion des Markierungsmusters und Wiederholung der Schritte a) und b) mit weiteren Reagenzlösungen Yn (n= 2, 3...N), die jeweils das mindestens eine Markiermolekül und/oder mindestens ein anderes Markiermolekül aufweisen;
d) Zusammenfassen der jeweils in Schritt b) detektierten Markierungsmuster zu einem komplexen molekularen Kombinationsmuster des Objekts X1;
e) Wiederholung der Schritte a) bis d) mit mindestens einem weiteren Objekt Xn (n= 2, 3...N), das andere Zellen und/oder andere Zellmembranen aufweist, die einer anderen Zell- oder Gewebeprobe entstammen;
f) Ermitteln mindestens eines Unterschieds zwischen dem Kombinationsmuster des Objektes X1 und dem Kombinationsmuster des Objektes Xn;
g) Identifizierung mindestens einer Reagenzlösung Y1 oder Yn, deren Markierungsmuster den in Schritt f) ermittelten Unterschied verursacht;
h) Selektion von Molekülen oder Molekülkomplexen, die durch das mindestens eine Markiermolekül der in Schritt g) identifizierten Reagenzlösung Y1 oder Yn gebunden werden, aus einem Homogenat von Zellen und/oder Zellmembranen, die der Zell- oder Gewebeprobe des sich gemäß Schritt f) unterscheidenden Objektes Xn entstammen; und
i) biochemisches Charakterisieren der gemäß Schritt h) selektierten Moleküle oder Molekülkomplexe.

2. Verfahren nach Anspruch 1,
**dadurch gekennzeichnet,**
**daß** das in Schritt h) verwendete Homogenat vor Schritt h) durch Molekül- oder Molekülkomplextrennverfahren, insbesondere Proteintrennverfahren, in einzelne Homogenatbestandteile aufgetrennt wird.

3. Verfahren nach einem der vorhergehenden Ansprüche,
**dadurch gekennzeichnet,**
**daß** das Objekt X1 Zellen und/oder Zellmembranen aufweist, die einer Zell- oder Gewebeprobe eines kranken Patienten entstammen, und daß mindestens ein anderes Objekt Xn Zellen und/oder Zellmembranen aufweist, die einer Zell- oder Gewebeprobe eines gesunden Probanden entstammen.

4. Verfahren nach einem der vorhergehenden Ansprüche,
**dadurch gekennzeichnet,**
**daß** das Verfahren folgenden parallelen Schritt umfaßt:
x) Erstellen jeweils eines Proteinexpressionsprofils von jeweils einem Probenanteil der Zell- oder Gewebeproben, von denen Zellen und/oder Zellmembranen in die Objekte X1 und Xn eingehen, und
Vergleichen des dem Objekt X1 zuzuordnenden Proteinexpressionsprofils mit dem dem Objekt Xn zuzuordnenden Proteinexpressionsprofil, wobei mindestens ein Unterschied festgestellt wird.

5. Verfahren nach Anspruch 4,
**dadurch gekennzeichnet,**
**daß** das Verfahren nach Schritt x) folgenden Schritt umfaßt:
y) Überprüfen mindestens eines Proteins und/oder mindestens einer Proteinmodifikation, das oder die den in Schritt x) ermittelten Unterschied verursacht, bezüglich einer Bindung des mindestens einen Markiermoleküls der in Schritt g) identifizierten Reagenzlösung Y1 oder Yn.

6. Verfahren nach Anspruch 4,
**dadurch gekennzeichnet,**
**daß** mindestens ein in Schritt a) und/oder Schritt c) verwendetes Markiermolekül mindestens ein Protein und/oder mindestens eine Proteinmodifikation bindet, das oder die den in Schritt x) ermittelten Unterschied verursacht.

7. Verfahren nach einem der vorhergehenden Ansprüche
**dadurch gekennzeichnet,**
**daß** mindestens ein in Schritt a) und/oder Schritt c) verwendetes Markiermolekül Fluorochrom-konjugiert ist.

8. Verfahren nach einem der vorhergehenden Ansprüche,
**dadurch gekennzeichnet,**
**daß** mindestens ein in Schritt a) und/oder Schritt c) verwendetes Markiermolekül ein Antikörper ist.

9. Verfahren nach Anspruch 8,
**dadurch gekennzeichnet,**
**daß** der Antikörper einer Antikörperbibliothek entnommen wird, wobei die Antikörperbibliothek eine naive oder eine nicht-naive Antikörperbibliothek ist.

10. Verfahren nach einem der vorhergehenden Ansprüche,
**dadurch gekennzeichnet,**
**daß** mindestens ein in Schritt a) und/oder Schritt c) verwendetes Markiermolekül ein Ligand ist.

11. Verfahren nach Anspruch 10,
**dadurch gekennzeichnet,**
**daß** der Ligand einer Ligandenbibliothek entnommen wird, wobei die Ligandenbibliothek eine naive oder eine nicht-naive Ligandenbibliothek ist.

12. Verfahren nach einem der vorhergehenden Ansprüche,
**dadurch gekennzeichnet,**
**daß** nach dem Entfernen der Reagenzlösung gemäß Schritt c) ein Waschschritt folgt, in dem eine Waschlösung auf das Objekt X1 aufgebracht und nach einer vorbestimmten Zeit wieder entfernt wird.

13. Verfahren nach einem der vorhergehenden Ansprüche,
**dadurch gekennzeichnet,**
**daß** Schritt d) durch computergestützte Bildüberlagerungen erfolgt.

14. Verfahren nach einem der vorhergehenden Ansprüche,
**dadurch gekennzeichnet,**
**daß** das Verfahren beliebig oft wiederholbare Bleichungszyklen, insbesondere nach Schritt b), umfaßt.

## Claims

1. Method for identifying cell-specific target structures comprising the following steps
a) automated application of a reagent solution Y1 having at least one marker molecule, to an object X1 having cells and/or cell membranes derived from a cell or tissue sample;
b) reaction of the reagent solution Y1 and automated detection of at least one marker pattern of the object X1 labeled with the reagent solution Y1;
c) removing the reagent solution Y1 before or after detection of the marker pattern, and repeating the steps a) and b) with further reagent solutions Yn (n=2, 3, ...N) each having the at least one marker molecule and/or at least one other marker molecule;
d) combining the marker patterns each detected in step b) to a complex molecular combination pattern of the object X1;
e) repeating the steps a) to d) with at least one further object Xn (n=2, 3...N) having other cells and/or other cell membranes derived from another cell or tissue sample;
f) determining at least one difference between the combination pattern of the object X1 and the combination pattern of the object Xn;
g) identifying at least one reagent solution Y1 or Yn, the marker pattern of which causes the difference determined in step f);
h) selecting molecules or molecule complexes, which are bound by the at least one marker molecule of the reagent solution Y1 or Yn identified in step g), from a homogenate of cells and/or cell membranes derived from the cell or tissue sample of the object Xn differing according to step f); and
i) biochemically characterizing the molecules or molecule complexes selected according to step h).

2. Method according to claim 1,
**characterized in that**
the homogenate used in step h) is separated into individual homogenate constituents by molecule or molecule complex separation methods, especially protein separation methods, before step h).

3. Method according to any one of the preceding claims,
**characterized in that**
the object X1 has cells and/or cell membranes derived from a cell or tissue sample of a diseased patient, and that at least one other object Xn has cells and/or cell membranes derived from a cell or tissue sample of a healthy subject.

4. Method according to any one of the preceding claims,
**characterized in that**
the method comprises the following parallel step:
x) creating a protein expression profile, respectively, of each one sample portion of the cell or tissue samples, from which cells and/or cell membranes enter into the objects X1 and Xn, and
comparing the protein expression profile to be associated with the object X1 with the protein expression profile to be associated with the object Xn, wherein at least one difference is determined.

5. Method according to claim 4,
**characterized in that**
after step x), the method comprises the following step:
y) checking at least one protein and/or at least one protein modification causing the difference determined in step x), with respect to a bond of the at least one marker molecule of the reagent solution Y1 or Yn identified in step g).

6. Method according to claim 4,
**characterized in that**
at least one marker molecule used in step a) and/or step c) binds at least one protein and/or at least one protein modification causing the difference determined in step x).

7. Method according to any one of the preceding claims,
**characterized in that**
at least one marker molecule used in step a) and/or step c) is fluorochromeconjugated.

8. Method according to any one of the preceding claims,
**characterized in that**
at least one marker molecule used in step a) and/or step c) is an antibody.

9. Method according to claim 8,
**characterized in that**
the antibody is extracted from an antibody library, wherein the antibody library is a naive or a non-naive antibody library.

10. Method according to any one of the preceding claims,
**characterized in that**
at least one marker molecule used in step a) and/or step c) is a ligand.

11. Method according to claim 10,
**characterized in that**
the ligand is extracted from a ligand library, wherein the ligand library is a naive or a non-naive ligand library.

12. Method according to any one of the preceding claims,
**characterized in that**
after removing the reagent solution according to step c), a washing step follows, in which a washing solution is applied to the object X1 and is removed again after a predetermined time.

13. Method according to any one of the preceding claims,
**characterized in that**
step d) is effected by computer-assisted image superpositions.

14. Method according to any one of the preceding claims,
**characterized in that**
the method includes bleaching cycles, which are repeatable any number of times, especially after step b).

## Revendications

1. Procédé d'identification de structures cellulaires cibles, comprenant les étapes suivantes:
a) application automatisée d'une solution de réactif Y1 comportant au moins une molécule de marquage sur un objet X1 comportant des cellules et/ou des membranes cellulaires originaires d'un échantillon de cellule ou de tissu;
b) imprégnation de la solution de réactif Y1 et détection automatisée d'au moins un modèle de marquage de l'objet X1 marqué par la solution de réactif Y1;
c) évacuation de la solution de réactif Y1 avant ou après détection du modèle de marquage et réitération des étapes a) et b) avec d'autres solutions de réactif Yn (n= 2, 3...N) comportant chacune l'une molécule de marquage au moins et/ou une autre molécule de marquage au moins;
d) regroupement des modèles de marquage détectés respectivement dans les étapes b) à un modèle de combinaison moléculaire complexe de l'objet X1;
e) réitération des étapes a) à d) avec au moins un objet supplémentaire Xn (n = 2, 3...N) comportant d'autres cellules et/ou d'autres membranes cellulaires originaires d'un autre échantillon de cellule ou de tissu;
f) détermination d'au moins une différence entre le modèle de combinaison de l'objet X1 et le modèle de combinaison de l'objet Xn;
g) identification d'au moins une solution de réactif Y1 ou Yn dont le modèle de marquage est à l'origine de la différence constatée dans l'étape f);
h) sélection de molécules ou de complexes moléculaires liés par l'au moins une molécule de marquage de la solution de réactif Y1 ou Yn identifiée dans l'étape g) sur un homogénat de cellules et/ou de membranes cellulaires provenant de l'échantillon de cellule ou de tissu de l'objet Xn se distinguant selon l'étape f); et
i) caractérisation biochimique des molécules ou complexes moléculaires sélectionnés selon l'étape h).

2. Procédé selon la revendication 1,
**caractérisé en ce que**
l'homogénat utilisé dans l'étape h) est séparé, avant l'étape h), au moyen de procédés de séparation de molécules ou de complexes moléculaires, notamment de séparation protéique, dans des composants d'homogénat individuels.

3. Procédé selon l'une des revendications précédentes,
**caractérisé en ce que**
l'objet X1 comporte des cellules et/ou des membranes cellulaires originaires d'un échantillon de tissu d'un malade et qu'au moins un autre objet Xn conporte des cellules et/ou des membranes cellulaires originaires d'un échantillon de tissu d'un sujet d'expérience non pas malade.

4. Procédé selon l'une des revendications précédentes,
**caractérisé en ce**
**que** le procédé comprend l'étape parallèle suivante:
x) établissement d'un profil d'expression protéique chacun d'une partie chacune des échantillons de cellule ou de tissu dont des cellules et/ou des membranes cellulaires entrent dans les objets X1 et Xn et
comparaison du profil d'expression protéique à associer à l'objet X1 au profil d'expression protéique à associer à l'objet Xn, procédure dans laquelle une différence au moins est retenue.

5. Procédé selon la revendication 4,
**caractérisé en ce que** le procédé comprend, après l'étape x), l'étape suivante:
y) vérification d'au moins une protéine et/ou d'au moins une modification protéique à l'origine de la différence constatée dans l'étape x) par rapport à la liaison de l'au moins une molécule de marquage de la solution de réactif Y1 ou Yn identifiée dans l'étape g).

6. Procédé selon la revendication 4,
**caractérisé en ce**
**qu'**au moins une molécule de marquage utilisée dans l'étape a) et/ou dans l'étape c) se combine avec au moins une protéine et/ou au moins une modification protéique étant à l'origine de la différence constatée dans l'étape x).

7. Procédé selon l'une des revendications précédentes,
**caractérisé en ce**
**qu'**au moins une molécule de marquage utilisée dans l'étape a) et/ou dans l'étape c) est conjuguée au fluorochrome.

8. Procédé selon l'une des revendications précédentes,
**caractérisé en ce**
**qu'**au moins une molécule de marquage utilisée dans l'étape a) et/ou dans l'étape c) est un anticorps.

9. Procédé selon la revendication 8,
**caractérisé en ce que** l'anticorps est prélevé sur une bibliothèque d'anticorps, celle-ci étant une bibliothèque d'anticorps naïve ou non.

10. Procédé selon l'une des revendications précédentes,
**caractérisé en ce**
**qu'**au moins une molécule de marquage utilisée dans l'étape a) et/ou dans l'étape c) est un ligand.

11. Procédé selon la revendication 10,
**caractérisé en ce**
**que** le ligand est prélevé sur une bibliothèque de ligands, celle-ci étant une bibliothèque de ligands naïve ou non.

12. Procédé selon l'une des revendications précédentes,
**caractérisé en ce**
**que** l'évacuation de la solution de réactif selon l'étape c) est suivie par une étape de lavage dans laquelle une solution de lavage est appliquée sur l'objet X1 pour être évacuée après une période prédéfinie.

13. Procédé selon l'une des revendications précédentes,
**caractérisé en ce que**
l'étape d) se fait grâce à des superpositions d'images assistées par ordinateur.

14. Procédé selon l'une des revendications précédentes,
**caractérisé en ce que**
le procédé comprend des cycles de blanchiment reproductibles autant de fois que l'on veut, notamment après l'étape b).
